# EUROPEAN PATENT APPLICATION

(11) **EP 3 888 697 A1**
(43) Date of publication of application: **06.10.2021**
(21) Application number: 20196448.3
(22) Date of filing: 16.09.2020
(51) Int. Cl.: A61L 2/10, A61L 2/22, A61L 9/14, A61L 9/20, B60H 3/00

(54) **DISINFECTION AND STERILIZATION APPARATUS AND METHOD, AND DISINFECTION AND STERILIZATION CONTROL APPARATUS**

(30) Priority: 31.03.2020 CN 202010245134
(71) Applicant: Beijing Xiaomi Mobile Software Co., Ltd., Beijing 100085 (CN)
(72) Inventor: XIE, Yingchun, Beijing 100085 (CN); XING, Zheng, Beijing 100085 (CN)
(74) Representative: dompatent von Kreisler Selting Werner - Partnerschaft von Patent- und Rechtsanwälten mbB

(57) **Abstract**

A disinfection and sterilization control apparatus (100) includes a disinfection and sterilization control component (110) configured to obtain usage data of a space; and a disinfection and sterilization component (120) electrically coupled to the disinfection and sterilization control component and configured to disinfect and sterilize the space under the control. The disinfection and sterilization control component controls the disinfection and sterilization component to perform disinfection and sterilization operation when the usage data meets a condition of triggering a disinfection and sterilization operation. When the usage data of the space meets the condition, the disinfection and sterilization apparatus is controlled to automatically disinfect and sterilize the space.

## Description

### BACKGROUND

Elevators are widely employed in buildings having more than one floors. Elevator cars are generally relatively small, can be densely packed with riders, and often are poorly ventilated. As such, elevator spaces may become places for bacteria and virus to breed and spread.

### SUMMARY

The present disclosure relates generally to elevator technologies, and more specifically to an elevator disinfection and sterilization control apparatus, an elevator disinfection and sterilization method, and an elevator disinfection and sterilization apparatus.

According to a first aspect of the embodiments of the present disclosure, an elevator disinfection and sterilization apparatus is provided. The elevator disinfection and sterilization apparatus includes a disinfection and sterilization control component and a disinfection and sterilization component. The disinfection and sterilization control component is configured to obtain usage data of an elevator and control the disinfection and sterilization component to perform disinfection and sterilization operation when the usage data meets a condition of triggering the disinfection and sterilization operation. The disinfection and sterilization component is electrically coupled to the disinfection and sterilization control component, and is configured to disinfect and sterilize the elevator under control of the disinfection and sterilization control component.

According to a second aspect of the embodiments of the present disclosure, an elevator disinfection and sterilization method is provided. The elevator disinfection and sterilization method includes: obtaining usage data of an elevator, and performing disinfection and sterilization operation on the elevator when the usage data meets a condition of triggering the disinfection and sterilization operation.

According to another aspect of the embodiments of the present disclosure, an elevator disinfection and sterilization control apparatus is provided, including: a processor, and a memory configured to store instructions executable by the processor. The processor is configured to execute the elevator disinfection and sterilization control method according to the second aspect of the embodiments of the present disclosure by reading the instructions stored in the memory.

It is to be understood that both the foregoing general description and the following detailed description are only exemplary and explanatory and are not restrictive of the present disclosure, as claimed.

### BRIEF DESCRIPTION OF THE DRAWINGS

The accompanying drawings, which are incorporated in and constitute a part of this disclosure, illustrate embodiments consistent with the present disclosure and, together with the description, serve to explain principles various embodiments of the present disclosure.
FIG. 1 is a schematic diagram of an elevator disinfection and sterilization control apparatus illustrated according to some embodiments of the present disclosure.
FIG. 2 is a flowchart of an elevator disinfection and sterilization control method illustrated according to some embodiments of the present disclosure.
FIG. 3 is a flowchart of an elevator disinfection and sterilization control method illustrated according to some other embodiments of the present disclosure.
FIG. 4 is a flowchart of an elevator disinfection and sterilization control method illustrated according to some other embodiments of the present disclosure.
FIG. 5 is a schematic diagram of an elevator disinfection and sterilization control method illustrated according to some embodiments of the present disclosure.
FIG. 6 is a block diagram of an apparatus illustrated according to some embodiments.

### DETAILED DESCRIPTION

Example embodiments will be explained in detail here, examples of which are illustrated in the accompanying drawings. The following description refers to the accompanying drawings in which the same numbers in different drawings represent the same or similar elements unless otherwise represented. The implementations set forth in the following description of example embodiments do not represent all implementations consistent with the present disclosure. Instead, they are merely examples of apparatuses and methods consistent with some aspects related to the present disclosure as detailed in the appended claims.

Currently, most of elevators are cleaned relying on cleaning staff to clean off the elevator cars and perform disinfection and sterilization on them. However, the cleaning time is not fixed and the cleaning effect is not ideal, which cannot guarantee the health of people. Alternatively, ventilation facilities may be installed in the elevator car to purify the air, but the virus and bacteria cannot be effectively removed from the car.

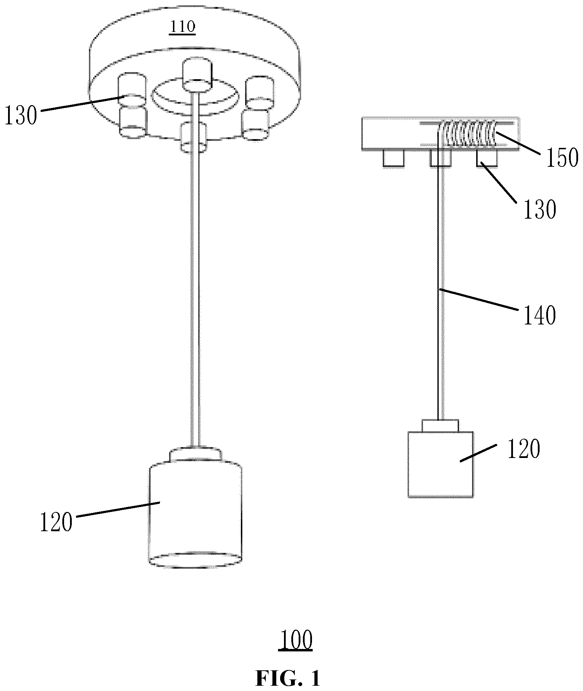

FIG. 1 is a schematic diagram of an elevator disinfection and sterilization control apparatus illustrated according to some embodiments of the present disclosure. The elevator disinfection and sterilization control apparatus 100 includes a disinfection and sterilization control component 110 and a disinfection and sterilization component 120.

The disinfection and sterilization control component 110 is configured to obtain usage data of the elevator and control the disinfection and sterilization component 120 to perform sterilization operation when the usage data meets a condition of triggering the sterilization operation.

The disinfection and sterilization component 120 is electrically coupled to the disinfection and sterilization control component 110, and is configured to disinfect and sterilize the elevator under control of the disinfection and sterilization control component 110.

As illustrated in FIG. 1, in some embodiments, the elevator disinfection and sterilization control apparatus 100 further includes a human body sensing component 130.

The human body sensing component 130 is disposed in the elevator, preferably on top of an elevator car, electrically coupled to the disinfection and sterilization control component 110, and configured to obtain a passenger carrying state of the elevator.

The human body sensing component 130 may be disposed at the base of the disinfection and sterilization control apparatus 100, for example, an infrared sensing device, for accurately sensing whether passengers, animals or goods are present in the elevator in real time. The disinfection and sterilization control component 110 uses the passenger carrying state of the elevator obtained by the human body sensing component 130 to determine whether the current elevator meets the work conditions of disinfection and sterilization the elevator. When the state of the elevator is no-load, the disinfection and sterilization component 120 is controlled to disinfect and sterilize the elevator.

As illustrated in FIG. 1, in some embodiments, the elevator disinfection and sterilization control apparatus 100 further includes a spool 140 and a hose 150. One end of the hose 150 therein is fixed on top of the elevator car, and the other end is connected to the disinfection and sterilization component 120, and an electrical connection line between the disinfection and sterilization component 120 and the disinfection and sterilization control component 110 is located inside the hose 150.

The spool 140 is fixed on top of the elevator car, the hose 150 is wound on the spool 140, and the spool 140 is wound under control of the disinfection and sterilization control component 110 to determine a position of the disinfection and sterilization component 120 in a height direction of the elevator car.

The spool 140 is wound under control of the disinfection and sterilization control component 110 to drive the disinfection and sterilization component 120 connected with the hose 150 and reciprocate along a vertical direction in the elevator car, thereby enabling the disinfection and sterilization of the disinfection and sterilization component 120 on the device at different heights.

The disinfection and sterilization control component 110 controls the winding of the spool 140, so as to control the disinfection and sterilization component 120 to stay at a specified height position of the elevator car, for example, the height of buttons or handrails disposed inside the elevator car. The disinfection and sterilization component 120 may perform a disinfection and sterilization operation at a specified height for a preset duration, so as to concentrate the disinfection and sterilization on key parts of the elevator and make the disinfection and sterilization more effective.

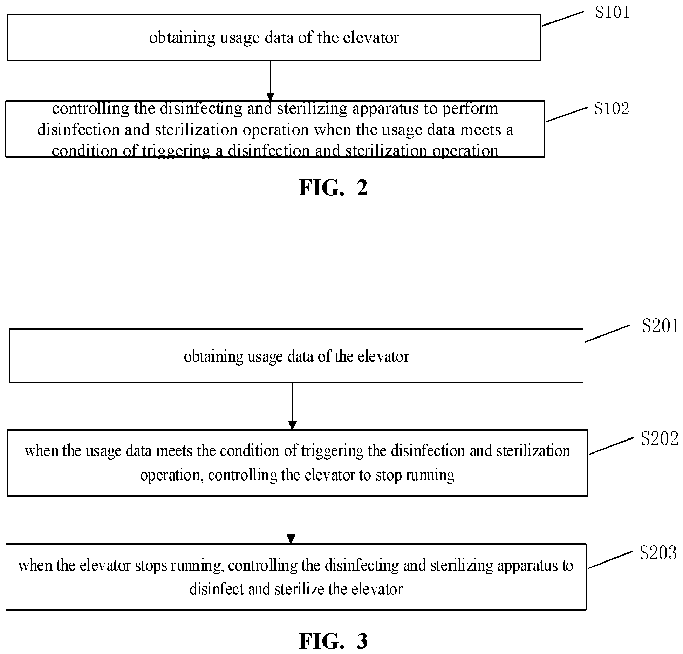

FIG. 2 is a flowchart of an elevator disinfection and sterilization control method illustrated according to some embodiments of the present disclosure. As illustrated in FIG. 2, the elevator includes a disinfection and sterilization apparatus, which may be the disinfection and sterilization component 120 shown in FIG. 1. The elevator disinfection and sterilization control method includes the following steps.

At step S101, usage data of an elevator is obtained by human body sensing component .

Elevator usage data may be obtained directly, or may be obtained from the elevator usage data provided by other intelligent devices connected through networks in the elevator.

At step S102, a disinfection and sterilization component is controlled by disinfection and sterilization control component to perform disinfection and sterilization operation on the elevator when the usage data meets a condition of triggering the disinfection and sterilization operation.

The elevator usage data may be used to characterize operation state and service state of the elevator. The elevator usage data is used as a condition of triggering the disinfection and sterilization operation on the elevator. When the elevator usage data meets the condition of triggering the disinfection and sterilization operation, the disinfection and sterilization control apparatus is controlled to perform disinfection and sterilization operation on the elevator.

According to embodiments of the present disclosure, when the usage data of the elevator meets the condition of triggering the disinfection and sterilization operation, the disinfection and sterilization control apparatus is controlled to automatically disinfect and sterilize the elevator, thereby realizing intelligent and effective disinfection and sterilization on the interior space of the elevator car, thereby avoiding cross infection, ensuring the safety of the elevator, and protecting the health of users.

In some embodiments, meeting the condition of triggering the disinfection and sterilization operation includes: the usage data of the elevator reaching a set threshold of usage data and/or a passenger carrying state of the elevator being no-load.

The elevator usage data includes service time of the elevator and/or the number of passengers taking the elevator. Meeting the condition of triggering the disinfection and sterilization operation may be that the service time of the elevator reaches the set threshold of elevator service time and/or the number of passengers taking the elevator reaches the set threshold of elevator passenger number.

The threshold of elevator service time and the threshold of elevator passenger number may be customized by the users themselves according to the specifications of the elevator and the importance of the elevator. For example, in public buildings such as hospitals, transportation hubs, shopping malls, and schools, the use frequency of elevator is high due to the large flow of people, and the possibility of pollution in elevators is high. The threshold of elevator service time and the threshold of elevator passenger number can be set to small values accordingly to increase the frequency of disinfection and sterilization operation and ensure the safety and hygiene of the elevator environment.

If there are passengers in the elevator, the disinfection and sterilization operation on the elevator may do damage to the passengers; and meanwhile, the passengers also affect the effect of the disinfection and sterilization operation. Therefore, meeting the condition of triggering the disinfection and sterilization operation includes that the passenger carrying state of the elevator is no-load.

It may be understood that the user can also actively disinfect and sterilize the no-load elevator as required even if the usage data of the elevator does not reach the set threshold of usage data, for example, during the period when elevator traffic is low, the usage data of the elevator does not reach the set threshold of usage data, the elevator is actively controlled for disinfection and sterilization. Thus this may avoid that the disinfection and sterilization operation on the elevator during the peak period of the elevator affects running of the elevator and user experience.

In some embodiments, the elevator includes a human body sensing component, and the method further includes: obtaining the passenger carrying state of the elevator by the human body sensing component.

The human body sensing component may be included in the elevator, and the human body sensing component may be disposed on the inner top of the elevator car, for example, an infrared sensing device, for accurately sensing whether passengers, animals or goods are present in the elevator in real time. The human body sensing component may obtain the passenger carrying state of the elevator in real time, and provide guarantee for the subsequent judgment on whether the conditions of triggering the disinfection and sterilization operation is met.

FIG. 3 is a flowchart of an elevator disinfection and sterilization method illustrated according to some other embodiments of the present disclosure. As illustrated in FIG. 3, the elevator disinfection and sterilization method includes the following steps.

At step S201, usage data of an elevator is obtained by human body sensing component.

At step S202, when the usage data meets the condition of triggering the disinfection and sterilization operation, the elevator is controlled to stop running.

When the usage data meets the condition of triggering the disinfection and sterilization operation, i.e., when the disinfection and sterilization operation on the elevator is determined, the elevator is controlled to stop running, and the elevator is remained in an off state.

At step S203, when the elevator stops running, the disinfection and sterilization operation is performed on the elevator by disinfection and sterilization component.

By carrying out the disinfection and sterilization operation on the elevator when it stops running, the impact of the disinfection and sterilization operation on the operating facilities of the elevator is avoided, and users or animals are prevented from entering into the elevator by mistake to affect the disinfection and sterilization operation, or the disinfecting means are avoided to cause damage to the users or animals.

In some embodiments, the disinfection and sterilization apparatus includes an ultraviolet emitting device and/or a medicinal spray device, and the ultraviolet emitting device is controlled to irradiate an interior space of the elevator car and/or the medicinal spray device is controlled to spray disinfectants to the interior space of the elevator car.

Referring to the disinfection and sterilization apparatus 130 in FIG. 2, the disinfection and sterilization apparatus 130 includes an ultraviolet emitting device. The ultraviolet emitting device (e.g., an ultraviolet lamp) may be disposed along a bottom of the nozzle of the disinfection and sterilization apparatus, and may emit ultraviolet light downwardly. Ultraviolet disinfection and sterilization utilizes ultraviolet rays of appropriate wavelengths to kill microorganisms by radiation damage to microbial pathogens such as bacteria, viruses, spores and other pathogens and destroying the function of their nucleic acids, so as to achieve the purpose of disinfection and sterilization on the air inside the elevator, the car side walls and car floor by means of ultraviolet radiation for a certain period of time.

The disinfection and sterilization apparatus 130 also includes a medicinal spray device, through which the medicines with a function of disinfection and sterilization may be sprayed. The disinfection and sterilization medicines are sprayed in the interior space of the elevator car to disinfect and sterilize the air inside the elevator, the car side walls and/or car floor. The medicinal spray device may be disposed along the side of the spraying nozzle of the disinfection and sterilization apparatus, so as to spray the medicines on the buttons on the side wall of the elevator car, thereby effectively disinfection and sterilization the elevator control buttons. The medicinal spray device may be also disposed along the bottom of the spraying nozzle of the disinfection and sterilization apparatus to perform a medicinal disinfection on the floor of the elevator car.

It may be understood that, controlling the ultraviolet emitting device to irradiate the interior space of the elevator car and controlling the medicinal spray device to spray the disinfectants to the interior space of the elevator car may be performed respectively and separately, or both ultraviolet emission and spraying medicines may be performed simultaneously, or ultraviolet emission or the spraying of medicines may be controlled according to the preset starting and ending times.

In some embodiments, the disinfection and sterilization apparatus further includes a lifting device, and the disinfection and sterilization apparatus is controlled to reciprocate along a height direction of the elevator car under the driving of the lifting device for overall disinfection and sterilization. Controlling the disinfection and sterilization apparatus to disinfect and sterilize the elevator may refer to controlling the lifting device to drive the disinfection and sterilization apparatus and reciprocate along the height direction of the elevator car, thereby enabling the overall disinfection and sterilization on the device at different heights of the elevator car.

Controlling the disinfection and sterilization apparatus to disinfect and sterilize the elevator may also refer to controlling the disinfection and sterilization apparatus to stay at a specified height for a concentrated disinfection and sterilization. For example, the lifting device is controlled to drive the disinfection and sterilization apparatus to stay at a preset height, for example, at the height where buttons or handrails of the elevator car is located, to carry out the disinfection and sterilization operation for a preset time, thus realizing the concentrated and effective sterilization on key parts of the elevator.

It may be understood that, the disinfection and sterilization operation mode, in which the disinfection and sterilization apparatus is controlled to reciprocate in the height direction of the elevator car for overall disinfection and sterilization, or to stay at the specified height position for the concentrated disinfection and sterilization on the key parts, can be set according to the use scenarios of the elevator.

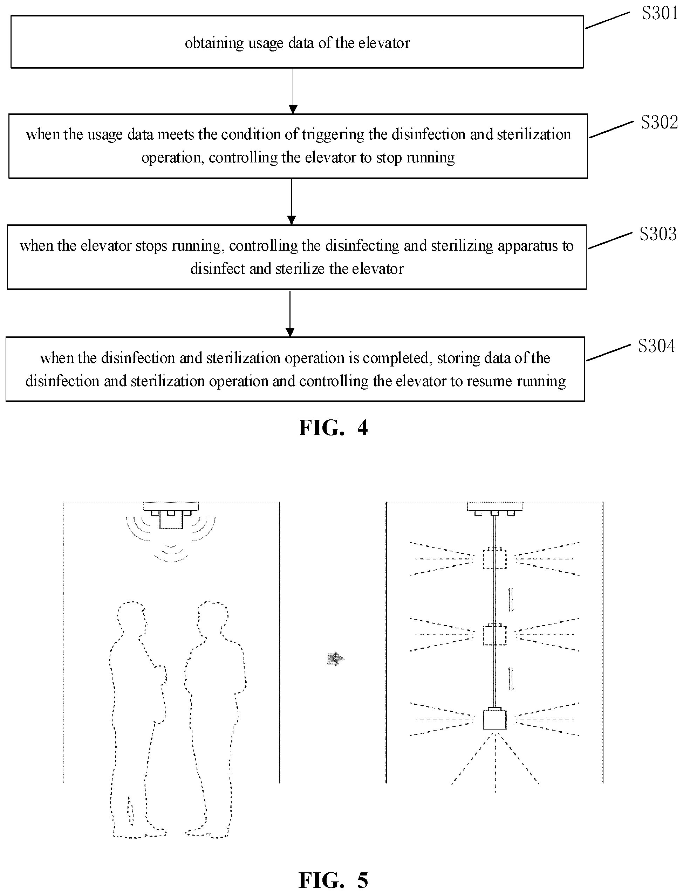

FIG. 4 is a flowchart of an elevator disinfection and sterilization control method illustrated according to some other embodiments of the present disclosure. As illustrated in FIG. 4, the elevator disinfection and sterilization control method includes the following steps.

At step S301, usage data of an elevator is obtained by human body sensing component.

At step S302, when the usage data meets the condition of triggering the disinfection and sterilization operation, the elevator is controlled to stop running.

At step S303, when the elevator stops running, the disinfection and sterilization operation is performed on the elevator by disinfection and sterilization component.

At step S304, when the disinfection and sterilization operation ends, the data of the disinfection and sterilization operation is saved and the elevator is controlled to resume running.

The elevator stops running (i.e., suspended) when the disinfection and sterilization operation is in progress. At the end of the disinfection and sterilization operation, the data of the disinfection and sterilization operation is saved, such as the starting time, duration, and operating mode of this disinfection and sterilization, for subsequent settings of the disinfection and sterilization operation again in order to achieve a reasonable and effective disinfection and sterilization of the elevator. At the end of the disinfection and sterilization operation, that is, after the disinfection and sterilization operation is completed, the elevator is controlled to resume running, and the elevator enters the manned working mode for users to take.

It may be understood that, in order to avoid bad experiences brought to the users by the disinfection and sterilization means, such as the unpleasant odor or harmful ingredients of the disinfectants used to affect the user experience, a time threshold may be set at the end of the disinfection and sterilization operation, and the interior space of the elevator car is ventilated to remove the adverse effects of the disinfection and sterilization means.

That is, only after the disinfection and sterilization operation is completed, the elevator enters the manned working mode. Meanwhile, the elevator backs up data of each disinfection and sterilization operation, and the disinfection and sterilization operation data is formed as working data and recorded in the network cloud backup. The elevator manager can remotely receive data to realize intelligent remote work.

In some embodiments, data of the disinfection and sterilization operation is saved in the network cloud.

The working data formed by the disinfection and sterilization operation is recorded in the network cloud backup, and the elevator manager can obtain the disinfection and sterilization operation data in the cloud to realize remote intelligent control of the elevator for disinfection and sterilization operation.

FIG. 5 is a schematic diagram of an elevator disinfection and sterilization control method illustrated according to some embodiments of the present disclosure. As illustrated in FIG. 5, the elevator obtains the passenger carrying state of the elevator through a sensing recognition device. When there is a living body inside the elevator, the condition of triggering the disinfection and sterilization operation are not satisfied. When the elevator determines that the elevator is no-load through the sensing recognition device, and/or the usage data of the elevator reaches the set threshold of usage data, the disinfection and sterilization apparatus is controlled to disinfect and sterilize the elevator.

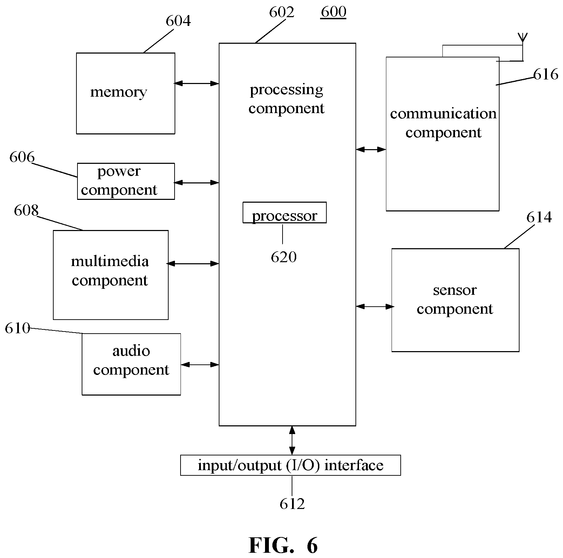

FIG. 6 is a block diagram of an apparatus illustrated according to an exemplary embodiment. For example, the elevator disinfection and sterilization control apparatus 600 may be a mobile phone, a computer, a digital broadcasting terminal, a messaging device, a game console, a tablet device, a medical device, a fitness device and a personal digital assistant.

As illustrated in FIG. 6, the elevator disinfection and sterilization control apparatus 600 may include one or more of the following components: a processing component 602, a memory 604, a power component 606, a multimedia component 608, an audio component 610, an input/output (I/O) interface 612, a sensor component 614, and a communication component 616.

The processing component 602 typically controls overall operations of the elevator disinfection and sterilization control apparatus 600, such as the operations associated with display, telephone calls, data communications, camera operations, and recording operations. The processing component 602 may include one or more processors 620 to execute instructions to perform all or part of the steps in the above described methods. Moreover, the processing component 602 may include one or more modules which facilitate the interaction between the processing component 602 and other components. For instance, the processing component 602 may include a multimedia module to facilitate the interaction between the multimedia component 608 and the processing component 602.

The memory 604 is configured to store various types of data to support the operation of the elevator disinfection and sterilization control apparatus 600. Examples of such data include instructions for any applications or methods operated on the elevator disinfection and sterilization control apparatus 600, contact data, phonebook data, messages, pictures, video, etc. The memory 604 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 606 provides power for various components of the elevator disinfection and sterilization control apparatus 600. The power component 606 may include a power management system, one or more power sources, and any other components associated with the generation, management, and distribution of power in the elevator disinfection and sterilization control apparatus 600.

The multimedia component 608 includes a screen providing an output interface between the elevator disinfection and sterilization control apparatus 600 and the user. In some embodiments, the screen may include a liquid crystal display (LCD) and a touch panel (TP). In some embodiments, an organic light-emitting diode (OLED) display or other types of displays may be employed.

If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a duration and a pressure associated with the touch or swipe action. In some embodiments, the multimedia component 608 includes a front camera and/or a rear camera. When the elevator disinfection and sterilization control apparatus 600 is in an operation mode, such as a shooting mode or a video mode, the front camera and/or the rear camera can receive external multimedia data. Each front camera and rear camera can be a fixed optical lens system or have focal length and optical zoom capabilities.

The audio component 610 is configured to output and/or input audio signals. For example, the audio component 610 includes a microphone ("MIC") configured to receive an external audio signal when the elevator disinfection and sterilization control apparatus 600 is in an operation mode, such as a call mode, a recording mode, and a voice recognition mode. The received audio signal may be further stored in the memory 604 or transmitted via the communication component 616. In some embodiments, the audio component 610 further includes a speaker for outputting audio signals.

The I/O interface 612 provides an interface between the processing component 602 and peripheral interface modules, such as a keyboard, a click wheel, buttons, and the like. The buttons may include, but are not limited to, a home button, a volume button, a starting button, and a locking button.

The sensor component 614 includes one or more sensors to provide status assessments on various aspects of the elevator disinfection and sterilization control apparatus 600. For instance, the sensor component 614 may detect an open/closed status of the elevator disinfection and sterilization control apparatus 600, and relative positioning of components, which are for example the display and the keypad, of the elevator disinfection and sterilization control apparatus 600. The sensor component 614 may also detect a change in position of the elevator disinfection and sterilization control apparatus 600 or a component of the elevator disinfection and sterilization control apparatus 600, a presence or absence of user contacting with the elevator disinfection and sterilization control apparatus 600, an orientation or an acceleration/deceleration of the elevator disinfection and sterilization control apparatus 600, and a change in temperature of the elevator disinfection and sterilization control apparatus 600. The sensor component 614 may include a proximity sensor configured to detect the presence of nearby objects without any physical contact. The sensor component 614 may also include a light sensor, such as a CMOS or CCD image sensor, for use in imaging applications. In some embodiments, the sensor component 614 may further include an acceleration sensor, a gyro sensor, a magnetic sensor, a pressure sensor, or a temperature sensor.

The communication component 616 is configured to facilitate communication, wired or wireless, between the elevator disinfection and sterilization control apparatus 600 and other devices. The elevator disinfection and sterilization control apparatus 600 can access a wireless network based on a communication standard, such as Wi-Fi, 2G, 3G, 4G, or 5G, or a combination thereof. In one example embodiment, the communication component 616 receives a broadcast signal or broadcast associated information from an external broadcast management system via a broadcast channel. In one example embodiment, the communication component 616 further includes a near field communication (NFC) module to facilitate short-range communications. For example, the NFC module may be implemented based on a radio frequency identity (RFID) technology, an infrared data association (IrDA) technology, an ultra-wideband (UWB) technology, a Bluetooth (BT) technology, and other technologi es.

In example embodiments, the elevator disinfection and sterilization control apparatus 600 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic elements, for performing the above described methods.

In example embodiments, there is also provided a non-transitory computer readable storage medium including instructions, such as included in the memory 604, executable by the processor in the elevator disinfection and sterilization control apparatus 600, for performing the above-described methods. For example, the non-transitory computer-readable storage medium may be a ROM, a RAM, a CD-ROM, a magnetic tape, a floppy disc, an optical data storage device, and the like.

Although various embodiments are described with respect to an elevator, some other embodiments of the present disclosure prove methods and apparatuses for use in other spaces, particularly high-usage public spaces that are closed and small. For example, the space can be an interior space of an elevator, a train car, an automobile, or an aircraft, and the disinfection and sterilization control component can be configured to control winding of a spool, so as to control the disinfection and sterilization component to automatically reach a specified height position of the interior space, to concentrate the disinfection and sterilization of high-touch areas including buttons or handrails inside the interior space, in addition to disinfecting and sterilizing the air and general surface areas of the interior space.

The various device components, modules, units, blocks, or portions may have modular configurations, or are composed of discrete components, but nonetheless can be referred to as rticularly high-usage public e, and the like.(BT) technology, and" "blocks," "portions," or "units" referred to herein may or may not be in modular forms, and these phrases may be interchangeably used.

In the present disclosure, the terms s, units, blocks, or portions may have modular configurations, or are composed of dly, and can be either a fixed connection or a detachable connection, or integrated, unless otherwise explicitly defined. These terms can refer to mechanical or electrical connections, or both. Such connections can be direct connections or indirect connections through an intermediate medium. These terms can also refer to the internal connections or the interactions between elements. The specific meanings of the above terms in the present disclosure can be understood by those of ordinary skill in the art on a case-by-case basis.

In the description of the present disclosure, the terms disclosure can be understood by those of ordinary skill in the art on a case-by-case basis.ed connection or a detachable connection, or integrated, unless otherwise explicitly deor example, a structure, a material or feature included in at least one embodiment or example. In the present disclosure, the schematic representation of the above terms is not necessarily directed to the same embodiment or example.

Moreover, the particular features, structures, materials, or characteristics described can be combined in a suitable manner in any one or more embodiments or examples. In addition, various embodiments or examples described in the specification, as well as features of various embodiments or examples, can be combined and reorganized.

Implementations of the subject matter and the operations described in this disclosure can be implemented in digital electronic circuitry, or in computer software, firmware, or hardware, including the structures disclosed herein and their structural equivalents, or in combinations of one or more of them. Implementations of the subject matter described in this disclosure can be implemented as one or more computer programs, i.e., one or more portions of computer program instructions, encoded on one or more computer storage medium for execution by, or to control the operation of, data processing apparatus.

Alternatively, or in addition, the program instructions can be encoded on an artificially-generated propagated signal, e.g., a machine-generated electrical, optical, or electromagnetic signal, which is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them.

Moreover, while a computer storage medium is not a propagated signal, a computer storage medium can be a source or destination of computer program instructions encoded in an artificially-generated propagated signal. The computer storage medium can also be, or be included in, one or more separate components or media (e.g., multiple CDs, disks, drives, or other storage devices). Accordingly, the computer storage medium can be tangible.

The operations described in this disclosure can be implemented as operations performed by a data processing apparatus on data stored on one or more computer-readable storage devices or received from other sources.

The devices in this disclosure can include special purpose logic circuitry, e.g., an FPGA (field-programmable gate array), or an ASIC (application-specific integrated circuit). The device can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The devices and execution environment can realize various different computing model infrastructures, such as web services, distributed computing, and grid computing infrastructures.

A computer program (also known as a program, software, software application, app, script, or code) can be written in any form of programming language, including compiled or interpreted languages, declarative or procedural languages, and it can be deployed in any form, including as a stand-alone program or as a portion, component, subroutine, object, or other portion suitable for use in a computing environment. A computer program can, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more portions, sub-programs, or portions of code). A computer program can be deployed to be executed on one computer or on multiple computers that are located at one site or distributed across multiple sites and interconnected by a communication network.

The processes and logic flows described in this disclosure can be performed by one or more programmable processors executing one or more computer programs to perform actions by operating on input data and generating output. The processes and logic flows can also be performed by, and apparatus can also be implemented as, special purpose logic circuitry, e.g., an FPGA, or an ASIC.

Processors or processing circuits suitable for the execution of a computer program include, by way of example, both general and special purpose microprocessors, and any one or more processors of any kind of digital computer. Generally, a processor will receive instructions and data from a read-only memory, or a random-access memory, or both. Elements of a computer can include a processor configured to perform actions in accordance with instructions and one or more memory devices for storing instructions and data.

Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few.

Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

To provide for interaction with a user, implementations of the subject matter described in this specification can be implemented with a computer and/or a display device, e.g., a VR/AR device, a head-mount display (HMD) device, a head-up display (HUD) device, smart eyewear (e.g., glasses), a CRT (cathode-ray tube), LCD (liquid-crystal display), OLED (organic light emitting diode), or any other monitor for displaying information to the user and a keyboard, a pointing device, e.g., a mouse, trackball, etc., or a touch screen, touch pad, etc., by which the user can provide input to the computer.

Implementations of the subject matter described in this specification can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the subject matter described in this specification, or any combination of one or more such back-end, middleware, or front-end components.

The components of the system can be interconnected by any form or medium of digital data communication, e.g., a communication network. Examples of communication networks include a local area network (component, e.g., an apnetwork (he components of the system can be interconnected by any form or medium of digital data communication, e.g., a

While this specification contains many specific implementation details, these should not be construed as limitations on the scope of any claims, but rather as descriptions of features specific to particular implementations. Certain features that are described in this specification in the context of separate implementations can also be implemented in combination in a single implementation. Conversely, various features that are described in the context of a single implementation can also be implemented in multiple implementations separately or in any suitable subcombination.

Moreover, although features can be described above as acting in certain combinations and even initially claimed as such, one or more features from a claimed combination can in some cases be excised from the combination, and the claimed combination can be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing can be advantageous. Moreover, the separation of various system components in the implementations described above should not be understood as requiring such separation in all implementations, and it should be understood that the described program components and systems can generally be integrated together in a single software product or packaged into multiple software products.

As such, particular implementations of the subject matter have been described. Other implementations are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking or parallel processing can be utilized.

It is intended that the specification and embodiments be considered as examples only. Other embodiments of the disclosure will be apparent to those skilled in the art in view of the specification and drawings of the present disclosure. That is, although specific embodiments have been described above in detail, the description is merely for purposes of illustration. It should be appreciated, therefore, that many aspects described above are not intended as required or essential elements unless explicitly stated otherwise.

Various modifications of, and equivalent acts corresponding to, the disclosed aspects of the example embodiments, in addition to those described above, can be made by a person of ordinary skill in the art, having the benefit of the present disclosure, without departing from the spirit and scope of the disclosure defined in the following claims, the scope of which is to be accorded the broadest interpretation so as to encompass such modifications and equivalent structures.

It should be understood that "a plurality" or correspoe" as referred to herein means two or more. "And/or," describing the association relationship of the associated objects, indicates that there may be three relationships, for example, A and/or B may indicate that there are three cases where A exists separately, A and B exist at the same time, and B exists separately. The character "/" generally indicates that the contextual objects are in an "or" relationship.

In the present disclosure, a first element being respoe" as referred to herein means two or more. "between the first and second elements, without contact, or indirect geometrical relationship through one or more intermediate media or layers, unless otherwise explicitly stated and defined. Similarly, a first element being r indirect bjects, indicates thah" a second element may indicate direct contact between the first and second elements, without contact, or indirect geometrical relationship through one or more intermediate media or layers, unless otherwise explicitly stated and defined.

Some other embodiments of the present disclosure can be available to those skilled in the art upon consideration of the specification and practice of the various embodiments disclosed herein. The present application is intended to cover any variations, uses, or adaptations of the present disclosure following general principles of the present disclosure and include the common general knowledge or conventional technical means in the art without departing from the present disclosure. The specification and examples can be shown as illustrative only, and the true scope and spirit of the disclosure are indicated by the following claims.

## Claims

1. A disinfection and sterilization apparatus (100), comprising a disinfection and sterilization control component (110) and a disinfection and sterilization component (120), wherein:
the disinfection and sterilization control component (120) is configured to obtain usage data of a space and control the disinfection and sterilization component to perform disinfection and sterilization operation when the usage data meets a condition of triggering the disinfection and sterilization operation; and
the disinfection and sterilization component (110) is electrically coupled with the disinfection and sterilization control component, and is configured to disinfect and sterilize the space under control of the disinfection and sterilization control component.

2. The disinfection and sterilization apparatus according to claim 1, further comprising:
a human body sensing component (130) disposed in the space, preferably on a ceiling of the space and electrically coupled with the disinfection and sterilization control component, and configured to obtain a passenger-carrying state of the space.

3. The disinfection and sterilization apparatus according to claim 1 or 2, further comprising:
a hose (150), wherein one end of the hose is fixed on a ceiling of the space, and the other end is connected with the disinfection and sterilization component, and an electrical connection line between the disinfection and sterilization component and the disinfection and sterilization control component is located inside the hose; and
a spool (140), wherein the spool is fixed to a ceiling of the space, the hose is wound on the spool, and the spool is wound under control of the disinfection and sterilization control component, so as to determine a position of the disinfection and sterilization component in a height direction of the space.

4. The disinfection and sterilization apparatus according to any one of claims 1-3, wherein the disinfection and sterilization component comprises an ultraviolet emitting device configured to disinfect and sterilize the space with ultraviolet irradiation.

5. The disinfection and sterilization apparatus according to any one of claims 1-4, wherein the disinfection and sterilization component comprises an injection orifice configured to spray disinfectants, and a reservoir configured to store the disinfectants.

6. The disinfection and sterilization apparatus according to claim 1, wherein the space is an interior space of an elevator, a train car, an automobile, or an aircraft, and the disinfection and sterilization control component is configured to control winding of a spool, so as to control the disinfection and sterilization component to automatically reach a specified height position of the interior space, to concentrate the disinfection and sterilization of high-touch areas including buttons or handrails inside the interior space.

7. A disinfection and sterilization method, comprising:
obtaining (S101; S201; S301) usage data of a space; and
performing (S102) disinfection and sterilization operation on the space when the usage data meets a condition of triggering the disinfection and sterilization operation.

8. The disinfection and sterilization method according to claim 7, wherein the condition of triggering the disinfection and sterilization operation comprises:
the usage data of the space reaching a set threshold of usage data and/or a passenger carrying state of the space being no-load, wherein the usage data comprises service time of the space and/or a number of users taking the space.

9. The disinfection and sterilization method according to claim 8, further comprising:
obtaining the passenger carrying state of the space.

10. The disinfection and sterilization method according to any one of claims 6-9, wherein the space is an enclosed space of an elevator, the method further comprising:
when the usage data meets the condition of triggering the disinfection and sterilization operation, controlling (S202; S302) the elevator to stop running; and
when the elevator stops running, performing (S203; S303) disinfection and sterilization operation on the elevator.

11. The disinfection and sterilization method according to any one of claims 6-10, wherein the performing disinfection and sterilization operation on the space comprises:
irradiating an interior space of the space with ultraviolet radiation; and/or
spraying disinfectants to the space.

12. The disinfection and sterilization method according to any one of claim 6-11, wherein the performing disinfection and sterilization operation in the space comprises:
performing overall disinfection and sterilization operation in the space; and
performing a concentrated disinfection and sterilization at a specified height position.

13. The disinfection and sterilization method according to any one of claims 6-12, wherein the space is an enclosed space of an elevator, the method further comprising:
when the disinfection and sterilization operation is completed, storing (S304) data of the disinfection and sterilization operation and controlling the elevator to resume running.

14. A disinfection and sterilization control apparatus, comprising:
a processor; and
a memory configured to store instructions executable by the processor;
wherein the processor is configured to execute a disinfection and sterilization control method according to any one of claims 6-13 by executing the instructions stored in the memory.
